# EUROPEAN PATENT SPECIFICATION

(11) **EP 2 794 023 B1**
(45) Date of publication and mention of the grant of the patent: **21.09.2022**
(21) Application number: 12806487.0
(22) Date of filing: 21.12.2012
(51) Int. Cl.: A61Q 5/06, A61K 8/06, A61K 8/36, A61K 8/37, A61K 8/891, A61K 8/31, A61K 8/39, A61K 8/894

(54) **HAIR TREATMENT WITH AN INVERSE EMULSION COMPRISING A DICARBONYL DERIVATIVE**
HAARBEHANDLUNGSVERFAHREN MIT EINER INVERSEN EMULSION ENTHALTEND EINE DICARBONYLVERBINDUNG
MÉTHODE DE TRAITEMENT DES CHEVEUX AVEC UNE ÉMULSION INVERSE COMPRENANT UN DÉRIVÉ DE DICARBONYLE

(30) Priority: 21.12.2011 FR 1162159; 31.01.2012 US 201261592963 P
(43) Date of publication of application: 29.10.2014
(73) Proprietor: L'Oréal, 75008 Paris (FR)
(72) Inventor: MORVAN, Christelle, F-95610 Eragny (FR)
(74) Representative: L'Oreal
(86) International application number: PCT/EP2012/076561
(87) International publication number: WO 2013/092959

(56) References cited:
- EP-A1- 0 416 969
- WO-A2-2011/104282
- US-A1- 2008 019 934

## Description

The present invention relates to an emulsion for treating human hair and keratin fibres, in the form of a water-in-oil inverse emulsion, containing a dicarbonyl compound.

Among the hair treatment methods for human keratin fibres, such as the hair, mention may be made of shampoos, hair conditioners, masks, lotions or creams, products for modifying the shape of the hair or its cosmetic properties, whether it is a matter of giving the hair a more wavy, curly or frizzy shape, or, conversely, of reducing its frizziness, curliness or waviness, and finally of controlling the consistency of curliness over time, under living conditions, heat and humidity. These modifications may be obtained over a short period, in the case of shampoos, especially treating shampoos or hair conditioners, or in a longer-lasting manner, in the case of permanent-waving or hair-straightening products.

More particularly, especially in the case of products for permanently modifying the shape, these treatments are based on the interaction between a treating agent present in an aqueous phase to which may be added either an oily phase or a solid fatty phase, the oily phase is then emulsified with surfactants in small amounts in the aqueous phase, and the solid fatty phase is in the form of a dispersion of fatty particles obtained by cooling an emulsion. This is famously the case for the α-hydroxy acids widely present in shampoos or hair conditioners, but also more recently in the hair-straightening aid products generally used as pretreatments before applying blow-drying (drying of the hair with a hairdryer concomitantly with the passage of a styling brush) or straightening irons brought to high temperature.

In order to improve the straightening or to obtain long-lasting improvement of the cosmetic properties of hair that may have been impaired by successive treatments or by exposure of the keratin fibres to their environment, it is known practice to use products often containing high concentrations of formol, or formaldehyde, or a form thereof, hydrate, polymer, methylol or adduct with amino acids. These treatments are particularly effective for ensuring long-lasting shaping of the hair, which allies movement, sheen, manageability, ease of styling and compatibility with all the other prior or subsequent treatments. Even the most damaged heads of hair can thus find a quality that is close to that of natural hair. However, the use of formaldehyde or derivatives thereof is regulated and limited to contents that are markedly below the amounts required for the efficiency of these treatments. Other techniques exist for aiding straightening using particular dicarbonyl compounds such as glyoxylic acid, or compounds of the type such as carboxymethylcysteine oxo acid acetamide, often in combination with mixtures of amino acids or keratin hydrolysates. Such products are described, for example, in patent applications WO-2007/135 299 and WO-2011/104 282. The compositions proposed according to this technique are concentrated acid solutions, generally having a very acidic pH, these compositions possibly containing liquid or solid fatty substances emulsified or dispersed with cationic surfactants.

Although having a relatively acceptable level of performance, these hair-straightening systems using glyoxylic acid or carboxymethylcysteine oxo acid acetamide require high concentrations of active compounds and strongly acidic pHs, which are not always well tolerated by the consumer.

Moreover, the use of compositions of very acidic pH give rise, during use, to undesired changes in the colour of natural hair, but also of artificially dyed or bleached hair.

Furthermore, the application of these compositions often leaves an unpleasant odour that may persist. There is thus still a need to develop straightening-aid products that can produce improved cosmetic properties.

This aim and others are achieved by the present invention, one subject of which is thus a process for treating human keratin fibres using a water-in-oil inverse emulsion comprising (a) at least 5% by weight of one or more liquid fatty substances relative to the total weight of the emulsion, (b) one or more surfactants and (c) one or more dicarbonyl compounds other than the compounds (a) which are an α-keto acid or a hydrate thereof, chosen from glyoxylic acid, pyruvic acid, oxaloacetic acid, ketoglutaric acid, in particular a hair shaping process such as a hair straightening process or a hair relaxing process.

The invention also relates to a water-in-oil inverse emulsion comprising (a) at least 5% by weight of one or more liquid fatty substances relative to the total weight of the emulsion, (b) one or more non-ionic surfactants and (c) one or more dicarbonyl compounds other than the compounds (a) which are an α-keto acid or a hydrate thereof, chosen from glyoxylic acid, pyruvic acid, oxaloacetic acid, ketoglutaric acid.

A subject of the invention is similarly a two-compartment device comprising, in one of the compartments, the inverse emulsion of the invention without dicarbonyl compounds according to c) and, in the other compartment, a composition containing one or more dicarbonyl compounds.

In the text hereinbelow, and unless otherwise indicated, the limits of a range of values are included in that range.

The expression "at least one" is equivalent to the expression "one or more".

The term "fatty substance" means an organic compound that is insoluble in water at ordinary temperature (25°C) and at atmospheric pressure (760 mmHg; i.e. 1.013×10⁵ Pa) (solubility of less than 5%, preferably of less than 1% and even more preferentially of less than 0.1%). They have, in their structure, at least one hydrocarbon-based chain comprising at least 6 carbon atoms or a sequence of at least two siloxane groups. In addition, the fatty substances are soluble in organic solvents under the same temperature and pressure conditions, for instance chloroform, ethanol or benzene.

The fatty substances in the context of the invention are liquid at room temperature (25°C) and at atmospheric pressure (780 mmHg). They are chosen especially from liquid fatty alcohols, liquid fatty esters, silicone oils, C₆-C₁₆ hydrocarbons, hydrocarbons containing more than 16 carbon atoms, non-silicone oils of animal origin, plant oils of triglyceride type, synthetic triglycerides, fluoro oils and non-salified fatty acids. These liquid fatty substances are neither polyoxyethylenated nor polyglycerolated.

The "liquid fatty alcohols", more generally known as "oils", are liquid at room temperature (25°C) and at atmospheric pressure.

The liquid fatty alcohols, in particular the C₁₀-C₃₄ alcohols, have branched carbon-based chains or contain one or more (preferably 1 to 3) unsaturations.

The liquid fatty alcohols according to the invention are preferably branched and/or unsaturated, and comprise from 12 to 40 carbon atoms. The fatty alcohols that are useful in the composition of the invention are non-oxyalkylenated and/or non-glycerolated.

The liquid fatty alcohols preferably have the structure R-OH, in which R preferably denotes a C₁₂-C₂₄ branched alkyl or C₁₂-C₂₄ alkenyl group. R may be substituted with one or more hydroxyl groups. Preferably, R does not contain any hydroxyl groups.

Examples that may be mentioned include oleyl alcohol, linoleyl alcohol, linolenyl alcohol, isocetyl alcohol, isostearyl alcohol, 2-octyl-1-dodecanol, 2-butyloctanol, 2-hexyl-1-decanol, 2-decyl-1-tetradecanol and 2-tetradecyl-1-cetanol, and mixtures thereof.

Preferably, the liquid fatty alcohol of the invention is a branched saturated alcohol. Even more preferentially, the liquid fatty alcohol of the invention is 2-octyl-1-dodecanol.

The fatty alcohols may be mixed, which means that several species may coexist in a commercial product, especially of different chain lengths, in the form of a mixture.

The liquid fatty esters that may be used in the present invention may be esters of monoalcohols or of polyols with monoacids or polyacids, at least one of the alcohols and/or acids comprising at least one chain of more than 7 carbon atoms. Preferably, the liquid fatty ester according to the invention is chosen from fatty acid esters of monoalcohols. Preferably, at least one of the alcohols and/or acids is branched.

Examples of liquid fatty esters according to the invention that may be mentioned include isopropyl myristate, isopropyl palmitate, isononyl isononanoate, 2-ethylhexyl palmitate, 2-hexyldecyl laurate, 2-octyldecyl palmitate and 2-octyldodecyl myristate.

The fatty acids that may be used as liquid fatty substances of the invention must not be salified in the composition, i.e. they must not be in the presence of a mineral or organic basifying agent such as sodium hydroxide, potassium hydroxide, monoethanolamine, triethanolamine or aqueous ammonia. As fatty acid that may be used, mention may be made of oleic acid.

The silicones that can be used in accordance with the invention are in the form of oils.

Preferably, the silicone is chosen from polydialkylsiloxanes, especially polydimethylsiloxanes (PDMS).

Organopolysiloxanes are defined in greater detail in Walter Noll's Chemistry and Technology of Silicones (1968), Academic Press. They may be volatile or non-volatile.

When they are volatile, the silicones are more particularly chosen from those with a boiling point of between 60°C and 260°C, and even more particularly from:
(i) cyclic polydialkylsiloxanes containing from 3 to 7 and preferably from 4 to 5 silicon atoms. These are, for example, octamethylcyclotetrasiloxane sold in particular under the name Volatile Silicone^{®} 7207 by Union Carbide or Silbione^{®} 70045 V 2 by Rhodia, decamethylcyclopentasiloxane sold under the name Volatile Silicone^{®} 7158 by Union Carbide, and Silbione^{®} 70045 V 5 by Rhodia, and mixtures thereof.

Mention may also be made of cyclocopolymers of the dimethylsiloxane/methylalkylsiloxane type, such as Volatile Silicone^{®} FZ 3109 sold by the company Union Carbide, of formula:

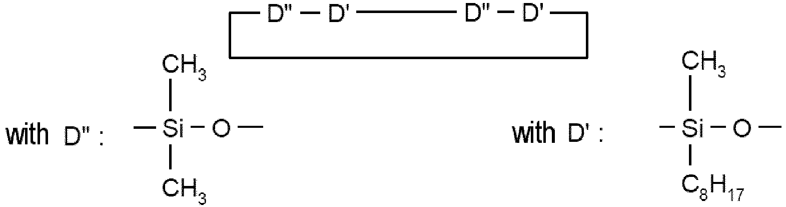

Mention may also be made of mixtures of cyclic polydialkylsiloxanes with organic compounds derived from silicon, such as the mixture of octamethylcyclotetrasiloxane and tetra(trimethylsilyl)pentaerythritol (50/50) and the mixture of octamethylcyclotetrasiloxane and oxy-1,1'-bis(2,2,2',2',3,3'-hexatrimethylsilyloxy)neopentane;
(ii) linear volatile polydialkylsiloxanes containing 2 to 9 silicon atoms and having a viscosity of less than or equal to 5×10⁻⁶ m²/s at 25°C. An example is decamethyltetrasiloxane sold in particular under the name SH 200 by the company Toray Silicone. Silicones belonging to this category are also described in the article published in Cosmetics and Toiletries, Vol. 91, Jan. 76, pp. 27-32, Todd & Byers, Volatile Silicone Fluids for Cosmetics*.*

Use is preferably made of non-volatile polydialkylsiloxanes, polyorganosiloxanes modified with the organofunctional groups above, and mixtures thereof.

These silicones are more particularly chosen from polydialkylsiloxanes, among which mention may be made mainly of polydimethylsiloxanes having trimethylsilyl end groups. The viscosity of the silicones is measured at 25°C according to ASTM standard 445 Appendix C.

Mention may be made, among these polydialkylsiloxanes, without implied limitation, of the following commercial products:
- the Silbione^{®} oils of the 47 and 70 047 series or the Mirasil^{®} oils sold by Rhodia, such as, for example, the oil 70 047 V 500 000;
- the oils of the Mirasil^{®} series sold by Rhodia;
- the oils of the 200 series from the company Dow Corning, such as DC200 with a viscosity of 60 000 mm²/s;
- the Viscasil^{®} oils from General Electric and certain oils of the SF series (SF 96, SF 18) from General Electric.

Mention may also be made of polydimethylsiloxanes having dimethylsilanol end groups known under the name of dimethiconol (CTFA), such as the oils of the 48 series from Rhodia.

In this category of polydialkylsiloxanes, mention may also be made of the products sold under the names Abil Wax^{®} 9800 and 9801 by the company Goldschmidt, which are poly(C₁-C₂₀)dialkylsiloxanes.

Products that can be used more particularly in accordance with the invention are mixtures such as:
- the mixtures formed from a hydroxy-terminated polydimethylsiloxane or dimethiconol (CTFA), and from a cyclic polydimethylsiloxane, also known as cyclomethicone (CTFA), such as the product Q2 1401 sold by Dow Corning;

The organomodified silicones that can be used in accordance with the invention are silicones as defined above and comprising in their structure one or more organofunctional groups attached via a hydrocarbon-based group.

In addition to the silicones described above, the organomodified silicones can be polydiarylsiloxanes, in particular polydiphenylsiloxanes, and polyalkylarylsiloxanes functionalized by the abovementioned organofunctional groups.

The polyalkylarylsiloxanes are chosen particularly from linear and/or branched polydimethyl/methylphenylsiloxanes and polydimethyl/diphenylsiloxanes with a viscosity of from 1 × 10⁻⁵ to 5×10⁻² m²/s at 25°C.

Among these polyalkylarylsiloxanes, examples that may be mentioned include the products sold under the following names:
- Silbione^{®} oils of the 70 641 series from Rhodia;
- oils of the Rhodorsil^{®} 70 633 and 763 series from Rhodia;
- the oil Dow Corning 556 Cosmetic Grade Fluid from Dow Corning;
- the silicones of the PK series from Bayer, such as the product PK20;
- the silicones of the PN and PH series from Bayer, such as the products PN1000 and PH1000;
- certain oils of the SF series from General Electric, such as SF 1023, SF 1154, SF 1250 and SF 1265.

Among the organomodified liquid silicones, mention may be made of polyorganosiloxanes comprising:
- substituted or unsubstituted amine groups,
- alkoxy groups.

Preferably, the silicones as liquid fatty substances are not organomodified.

As regards the C₆-C₁₆ hydrocarbons, they are linear or branched, and possibly cyclic, and are preferably alkanes. Examples that may be mentioned include hexane, dodecane and isoparaffins such as isohexadecane and isodecane.

Mention may be made, as hydrocarbon oils of animal origin, of perhydrosqualene.

The triglyceride oils of plant or synthetic origin are preferably chosen from liquid fatty acid triglycerides containing from 6 to 30 carbon atoms, for instance heptanoic or octanoic acid triglycerides, or alternatively, for example, sunflower oil, corn oil, soybean oil, marrow oil, grapeseed oil, sesame seed oil, hazelnut oil, apricot oil, macadamia oil, arara oil, castor oil, avocado oil, caprylic/capric acid triglycerides, for instance those sold by the company Stéarineries Dubois or those sold under the names Miglyol^{®} 810, 812 and 818 by the company Dynamit Nobel, jojoba oil and shea butter oil.

The linear or branched hydrocarbons of mineral or synthetic origin having more than 16 carbon atoms are preferably chosen from liquid paraffins, petrolatum, liquid petrolatum, polydecenes or hydrogenated polyisobutene, such as Parleam^{®}.

The fluoro oils may be chosen from perfluoromethylcyclopentane and perfluoro-1,3-dimethylcyclohexane, sold under the names Flutec^{®} PC1 and Flutec^{®} PC3 by the company BNFL Fluorochemicals; perfluoro-1,2-dimethyl-cyclobutane; perfluoroalkanes such as dodecafluoropentane and tetradecafluorohexane, sold under the names PF 5050^{®} and PF 5060^{®} by the company 3M, or bromoperfluorooctyl sold under the name Foralkyl^{®} by the company Atochem; nonafluoromethoxybutane and nonafluoroethoxyisobutane; perfluoromorpholine derivatives such as 4-trifluoromethyl perfluoromorpholine sold under the name PF 5052^{®} by the company 3M.

Preferably, the liquid fatty substance according to the invention is chosen from silicone oils and liquid fatty alcohols. Even more preferentially, the liquid fatty substance is chosen from silicone oils.

The human keratin fibres treated via the process according to the invention are preferably the hair.

The oil/water inverse emulsions according to the invention are true emulsions, and should be distinguished from microemulsions, which are thermodynamically stable systems, unlike true emulsions.

The size of the droplets of the dispersed phase of the emulsions of the invention is preferably between 10 nm and 100 µm and preferably between 200 nm and 50 µm. This is the mean diameter D(3.2), which may be measured especially using a laser particle sizer.

Preferably, the concentration of liquid fatty substances in the inverse emulsions of the invention may range from 5% to 90%, preferably from 7% to 50% and even more preferentially from 7% to 20% relative to the total weight of the emulsion.

The emulsions of the invention may comprise one or more other additional solid fatty substances.

More particularly, the additional fatty substances are chosen from hydrocarbons, non-silicone oils of plant or synthetic origin other than the liquid hydrocarbons, fatty alcohols, esters of fatty acids and/or of fatty alcohols, non-silicone waxes, and silicones other than the liquid silicones of the invention.

In particular, the composition of the invention may contain one or more fatty esters that are solid at room temperature (25°C) and at atmospheric pressure. Preferably, these solid fatty esters are esters of saturated carboxylic acids comprising at least 10 carbon atoms and of saturated fatty monoalcohols comprising at least 10 carbon atoms. The saturated acids or monoalcohols may be linear or branched. The saturated carboxylic acids preferably comprise from 10 to 30 carbon atoms and more preferentially from 12 to 24 carbon atoms. They may optionally be hydroxylated. The saturated fatty monoalcohols preferably comprise from 10 to 30 carbon atoms and more particularly from 12 to 24 carbon atoms.

Preferably, the solid fatty esters are chosen from myristyl myristate, cetyl myristate, stearyl myristate, myristyl palmitate, cetyl palmitate, stearyl palmitate, myristyl stearate, cetyl stearate and stearyl stearate, and mixtures thereof.

The compositions of the invention may contain one or more fatty alcohols that are solid at room temperature (25°C) and at atmospheric pressure. Preferably, the solid fatty alcohols are of structure R-OH with R denoting a linear alkyl group, optionally substituted with one or more hydroxyl groups, comprising from 12 to 40 and better still from 12 to 30 carbon atoms.

The fatty alcohols that are solid at room temperature, which are suitable for use in the invention, are more particularly chosen from:
- lauryl alcohol (1-dodecanol);
- myristyl alcohol (1-tetradecanol);
- cetyl alcohol (1-hexadecanol);
- stearyl alcohol (1-octadecanol);
- arachidyl alcohol (1-eicosanol);
- behenyl alcohol (1-docosanol);
- lignoceryl alcohol (1-tetracosanol);
- ceryl alcohol (1-hexacosanol);
- montanyl alcohol (1-octacosanol);
- myricyl alcohol (1-triacontanol);
and mixtures thereof.

More particularly, the solid fatty alcohol is chosen from cetyl alcohol, stearyl alcohol, behenyl alcohol and mixtures thereof such as cetylstearyl alcohol or cetearyl alcohol.

The inverse emulsion of the invention may also comprise silicones other than liquid silicones, for example silicones in the form of waxes, resins or gums.

Preferably, the non-liquid silicones are chosen from polydialkylsiloxanes, especially polydimethylsiloxanes (PDMS), and organomodified polysiloxanes comprising at least one functional group chosen from poly(oxyalkylene) groups, amino groups and alkoxy groups.

The silicone gums that may be used in accordance with the invention are especially polydialkylsiloxanes and preferably polydimethylsiloxanes with high number-average molecular weights of between 200 000 and 1 000 000, used alone or as a mixture in a solvent. These gums are, for example, mixtures such as:
- the mixtures formed from a hydroxy-terminated polydimethylsiloxane or dimethiconol (CTFA), and from a cyclic polydimethylsiloxane, also known as cyclomethicone (CTFA), such as the product Q2 1401 sold by Dow Corning;
- the mixtures of a polydimethylsiloxane gum and of a cyclic silicone, such as the product SF 1214 Silicone Fluid from General Electric; this product is an SF 30 gum corresponding to a dimethicone, having a number-average molecular weight of 500 000, dissolved in the oil SF 1202 Silicone Fluid corresponding to decamethylcyclopentasiloxane;
- the mixtures of two PDMSs with different viscosities, and more particularly of a PDMS gum and a PDMS oil, such as the product SF 1236 from General Electric. The product SF 1236 is a mixture of a gum SE 30 defined above, with a viscosity of 20 m²/s and of an oil SF 96 with a viscosity of 5×10⁻⁶ m²/s. This product preferably comprises 15% of gum SE 30 and 85% of an oil SF 96.

The organopolysiloxane resins that can be used in accordance with the invention are crosslinked siloxane systems containing the following units:

R₂SiO_{2/2}, R₃SiO_{1/2}, RSiO_{3/2} and SiO_{4/2},

in which R represents an alkyl containing 1 to 16 carbon atoms. Among these products, the ones that are particularly preferred are those in which R denotes a C₁-C₄ lower alkyl group, more particularly methyl.

Among these resins, mention may be made of the product sold under the name Dow Corning 593 or those sold under the names Silicone Fluid SS 4230 and SS 4267 by the company General Electric, which are silicones of dimethyl/trimethylsiloxane structure.

Mention may also be made of the trimethyl siloxysilicate type resins sold especially under the names X22-4914, X21-5034 and X21-5037 by the company Shin-Etsu.

Among the additional organomodified silicones, mention may be made of polyorganosiloxanes comprising:
- substituted or unsubstituted amine groups, for instance the products sold under the names Q2 8220 and Dow Corning 929 or 939 by the company Dow Corning. The substituted amine groups are, in particular, C₁-C₄ aminoalkyl groups;
- alkoxylated groups, such as the products sold under the names Abil Wax^{®} 2428, 2434 and 2440 by Goldschmidt.

The inverse emulsion according to the invention may have a content of additional fatty substances ranging from 0.1% to 40% by weight, even more preferentially from 0.5% to 25% by weight and better still from 1% to 20% by weight relative to the weight of the emulsion.

The emulsion of the invention comprises at least one dicarbonyl compound other than the compounds (a) which are an α-keto acid or a hydrate thereof, chosen from glyoxylic acid, pyruvic acid, oxaloacetic acid, ketoglutaric acid.

This agent is defined by the presence in its structure of at least two carbonyl groups (double bond C=O), at least one of which is not directly bonded to a hydroxyl group and is therefore other than a carboxylic acid function. This group is therefore an aldehyde, ketone, Preferably, this function is an aldehyde or ketone, and it is more preferentially an aldehyde.

Two carbonyl groups are bonded together either via a direct bond between the two carbon atoms, or via a linear or branched hydrocarbon-based chain, which may be saturated or unsaturated, and may be interrupted one or more times with a heteroatom such as nitrogen, in which case it bears either a hydrogen radical or an alkyl or acyl chain, oxygen, sulfur or a combination of these atoms. Preferably, the two carbonyl groups are bonded together via a direct bond between the two carbon atoms or alternatively an alkylene chain of formula (CH₂)ₙ for which the value of n is between 1 and 8; more preferentially, the two carbonyl groups are directly bonded together.

In the case where only one of the carbonyl groups is not a constituent of a carboxylic acid group but of an ester group, it is an alcohol ester, the said alcohol being optionally substituted with one or more hydroxyl or carboxyl groups. The unit derived from the alcohol preferably has a molecular mass of less than 100, and is preferably an ethyloxy, propyloxy, isopropyloxy or isobutyloxy group.

In the case where one of the carbonyl groups is not a constituent of a carboxylic acid group but of a keto group, the chain bonded to this keto group and linking at least one other carbonyl group is preferably a linear or branched C₁-C₈ hydrocarbon-based chain, optionally substituted with one or more hydroxyl groups.

In the case where two carbonyl groups are not carboxylic acid groups, they may be identical or different and may constitute, for example, a dialdehyde, a diketone or a diester, an aldehyde ester, a keto ester, a keto aldehyde, a keto amide or an amido aldehyde, the definitions of these side chains formed by the ester groups, on the one hand, and keto groups, on the other hand, corresponding to the definitions given, respectively, in the two preceding paragraphs.

Where appropriate, in the case where one of the carbonyl groups is a ketone or aldehyde group, this group may also be present in a masked form such as a hydrate, ketal or hemiketal form, or an amine form. Preferably, the dicarbonyl compound is chosen from α-keto acids and hydrates thereof.

The compounds thus defined are, for example, glyoxylic acid, pyruvic acid, oxaloacetic acid, ketoglutaric acid. Preferably, the dicarbonyl compound is chosen from α-keto acids and hydrates thereof.

Even more preferentially, the dicarbonyl compound is glyoxylic acid or a hydrate thereof, also known as oxoacetic acid (OCHCO₂H).

The inverse emulsion of the invention comprises one or more surfactants. Examples of surfactants that may be mentioned include anionic, amphoteric, zwitterionic, cationic or nonionic surfactants.

The term *"anionic surfactant"* means a surfactant comprising, as ionic or ionizable groups, only anionic groups. These anionic groups are preferably chosen from the groups -C(O)OH, -C(O)O⁻, -SO₃H, -S(O)₂O⁻, -OS(O)₂OH, -OS(O)₂O⁻, - P(O)OH₂, -P(O)₂O⁻, -P(O)O₂⁻, -P(OH)₂, =P(O)OH, -P(OH)O⁻, =P(O)O⁻, =POH and =PO⁻, the anionic parts comprising a cationic counterion such as an alkali metal, an alkaline-earth metal or an ammonium.

As examples of anionic surfactants that may be used in the composition mention may be made of alkyl sulfates, alkyl ether sulfates, alkylamido ether sulfates, alkylarylpolyether sulfates, monoglyceride sulfates, alkylsulfonates, alkylamidesulfonates, alkylarylsulfonates, α-olefin sulfonates, paraffin sulfonates, alkylsulfosuccinates, alkylether sulfosuccinates, alkylamide sulfosuccinates, alkylsulfoacetates, acylsarcosinates, acylglutamates, alkylsulfosuccinamates, acylisethionates and N-acyltaurates, salts of alkyl monoesters of polyglycoside-polycarboxylic acids, acyllactylates, D-galactoside uronic acid salts, alkyl ether carboxylic acid salts, alkylaryl ether carboxylic acid salts, alkylamido ether carboxylic acid salts; and the corresponding non-salified forms of all these compounds; the alkyl and acyl groups of all these compounds comprising from 6 to 40 carbon atoms and the aryl group denoting a phenyl group.

These compounds can be oxyethylenated and then preferably comprise from 1 to 50 ethylene oxide units.

The salts of C₆-C₂₄ alkyl monoesters of polyglycoside-polycarboxylic acids can be chosen from C₆-C₂₄ alkyl polyglycoside-citrates, C₆-C₂₄ alkyl polyglycoside-tartrates and C₆-C₂₄ alkyl polyglycoside-sulfosuccinates.

When the anionic surfactant(s) are in salt form, they may be chosen from alkali metal salts such as the sodium or potassium salt and preferably the sodium salt, ammonium salts, amine salts and in particular amino alcohol salts or alkaline-earth metal salts such as the magnesium salts.

Examples of amino alcohol salts that may especially be mentioned include monoethanolamine, diethanolamine and triethanolamine salts, monoisopropanolamine, diisopropanolamine or triisopropanolamine salts, 2-amino-2-methyl-1-propanol salts, 2-amino-2-methyl-1,3-propanediol salts and tris(hydroxymethyl)aminomethane salts.

Alkali metal or alkaline-earth metal salts, and in particular sodium or magnesium salts, are preferably used.

Use is preferably made, among the anionic surfactants mentioned, of (C₆-C₂₄)alkyl sulfates, (C₆-C₂₄)alkyl ether sulfates comprising from 2 to 50 ethylene oxide units, in particular in the form of alkali metal, ammonium, amino alcohol and alkaline-earth metal salts, or a mixture of these compounds.

In particular, it is preferable to use (C₁₂-C₂₀)alkyl sulfates, (C₁₂-C₂₀)alkyl ether sulfates comprising from 2 to 20 ethylene oxide units, in particular in the form of alkali metal, ammonium, amino alcohol and alkaline-earth metal salts, or a mixture of these compounds. Better still, it is preferred to use sodium lauryl ether sulfate containing 2.2 mol of ethylene oxide.

The amphoteric or zwitterionic surfactant(s) that may be used in the present invention are preferably non-silicone. They may especially be optionally quaternized secondary or tertiary aliphatic amine derivatives, in which the aliphatic group is a linear or branched chain comprising from 8 to 22 carbon atoms, the said amine derivatives containing at least one anionic group, for instance a carboxylate, sulfonate, sulfate, phosphate or phosphonate group. Mention may be made in particular of (C₈-C₂₀)alkylbetaines, sulfobetaines, (C₈-C₂₀)alkylamido(C₃-C₈)alkylbetaines and (C₈-C₂₀)alkylamido(C₆-C₈)alkylsulfobetaines.

Among the optionally quaternized secondary or tertiary aliphatic amine derivatives that can be used, as defined above, mention may also be made of the compounds of respective structures (A1) and (A2):

Rₐ-C(O)-NH-CH₂-CH₂-N⁺(R_{b})(R_{c})-CH₂C(O)O⁻, M⁺, X⁻ (A1)

in which formula (A1):
▪ Rₐ represents a C₁₀-C₃₀ alkyl or alkenyl group derived from an acid Rₐ-COOH preferably present in hydrolysed coconut oil, or a heptyl, nonyl or undecyl group;
▪ R_{b} represents a β-hydroxyethyl group; and
▪ R_{c} represents a carboxymethyl group;
▪ M⁺ represents a cationic counterion derived from an alkali metal or alkaline-earth metal, such as sodium, an ammonium ion or an ion derived from an organic amine, and
▪ X⁻ represents an organic or inorganic anionic counterion, such as that chosen from halides, acetates, phosphates, nitrates, (C₁-C₄)alkyl sulfates, (C₁-C₄)alkyl- or (C₁-C₄)alkylarylsulfonates, in particular methyl sulfate and ethyl sulfate; or alternatively M⁺ and X⁻ are absent;
▪

   R_{a'}-C(O)-NH-CH₂-CH₂-N(B)(B') (A2)

   in which formula (A2):
▪ B represents the group -CH₂-CH₂-O-X';
▪ B' represents the group -(CH₂)_{z}Y', with z = 1 or 2;
▪ X' represents the group -CH₂-C(O)OH, -CH₂-C(O)OZ', -CH₂-CH₂-C(O)OH, -CH₂-CH₂-C(O)OZ', or a hydrogen atom;
▪ Y' represents the group -C(O)OH, -C(O)OZ', -CH₂-CH(OH)-SO₃H or the group -CH₂-CH(OH)-SO₃-Z';
▪ Z' represents a cationic counterion derived from an alkali metal or alkaline-earth metal, such as sodium, an ammonium ion or an ion derived from an organic amine;
▪ R_{a'} represents a C₁₀-C₃₀ alkyl or alkenyl group of an acid R_{a'}-C(O)OH preferably present in coconut oil or in hydrolysed linseed oil, an alkyl group, especially of C₁₇ and its iso form, or an unsaturated C₁₇ group.

These compounds are classified in the CTFA dictionary, 5th edition, 1993, under the names disodium cocoamphodiacetate, disodium lauroamphodiacetate, disodium caprylamphodiacetate, disodium capryloamphodiacetate, disodium cocoamphodipropionate, disodium lauroamphodipropionate, disodium caprylamphodipropionate, disodium capryloamphodipropionate, lauroamphodipropionic acid and cocoamphodipropionic acid.

By way of example, mention may be made of the cocoamphodiacetate sold by the company Rhodia under the trade name Miranol^{®} C2M Concentrate.

Among the amphoteric or zwitterionic surfactants mentioned above, use is preferably made of (C₈-C₂₀)alkylbetaines such as cocoylbetaine, and (C₈-C₂₀)alkylamido(C₃-C₈)alkylbetaines such as cocamidopropylbetaine, and mixtures thereof. More preferentially, the amphoteric or zwitterionic surfactant(s) are chosen from cocamidopropylbetaine and cocoylbetaine.

The cationic surfactant(s) which can be used in the composition according to the invention comprise, for example, salts of optionally polyoxyalkylenated primary, secondary or tertiary fatty amines, quaternary ammonium salts, and mixtures thereof.

Examples of quaternary ammonium salts that may especially be mentioned include:
- those corresponding to the general formula (A3) below: 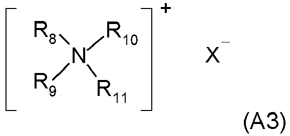 in which formula (A3):
   ▪ R8 to R11, which may be identical or different, represent a linear or branched aliphatic group comprising from 1 to 30 carbon atoms, or an aromatic group such as aryl or alkylaryl, it being understood that at least one of the groups R8 to R11 comprises from 8 to 30 carbon atoms and preferably from 12 to 24 carbon atoms; and
   ▪ X⁻ represents an organic or inorganic anionic counterion, such as that chosen from halides, acetates, phosphates, nitrates, (C₁-C₄)alkyl sulfates, (C₁-C₄)alkyl or (C₁-C₄)alkylaryl sulfonates, in particular methyl sulfate and ethyl sulfate.

The aliphatic groups of R8 to R11 may also comprise heteroatoms especially such as oxygen, nitrogen, sulfur and halogens.

The aliphatic groups of R8 to R11 are chosen, for example, from C₁-C₃₀ alkyl, C₁-C₃₀ alkoxy, polyoxy(C2-C6)alkylene, C₁-C₃₀ alkylamide, (C₁₂-C₂₂)alkylamido(C₂-C₆)alkyl, (C₁₂-C₂₂)alkyl acetate, C₁-C₃₀ hydroxyalkyl groups, X⁻ is an anionic counterion chosen from the group of halides, phosphates, acetates, lactates, (C₁-C₄)alkyl sulfates, and (C₁-C₄)alkyl- or (C₁-C₄)alkylarylsulfonates.

Among the quaternary ammonium salts of formula (A3), preference is given firstly to tetraalkylammonium chlorides, for instance dialkyldimethylammonium or alkyltrimethylammonium chlorides in which the alkyl group contains approximately from 12 to 22 carbon atoms, in particular behenyltrimethylammonium chloride, distearyldimethylammonium chloride, cetyltrimethylammonium chloride, benzyldimethylstearylammonium chloride, or else, secondly, distearoylethylhydroxyethylmethylammonium methosulfate, dipalmitoylethylhydroxyethylammonium methosulfate or distearoylethylhydroxyethylammonium methosulfate, or else, lastly, palmitylamidopropyltrimethylammonium chloride or stearamidopropyldimethyl(myristyl acetate)ammonium chloride, sold under the name Ceraphyl^{®} 70 by the company Van Dyk;
- quaternary ammonium salts of imidazoline, for instance those of formula (A4) below: 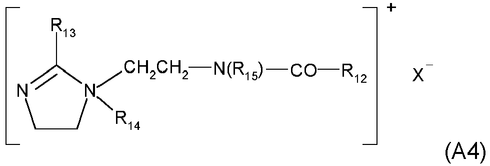 in which formula (A4):
   ▪ R12 represents an alkenyl or alkyl group comprising from 8 to 30 carbon atoms, for example tallow fatty acid derivatives;
   ▪ R13 represents a hydrogen atom, a C₁-C₄ alkyl radical or an alkenyl or alkyl radical containing from 8 to 30 carbon atoms;
   ▪ R14 represents a C₁-C₄ alkyl group;
   ▪ R15 represents a hydrogen atom or a C₁-C₄ alkyl group;
   ▪ X⁻ represents an organic or inorganic anionic counterion, such as that chosen from halides, phosphates, acetates, lactates, (C₁-C₄)alkyl sulfates, (C₁-C₄)alkyl or (C₁-C₄)alkylaryl sulfonates.
   R12 and R13 preferably denote a mixture of alkyl or alkenyl groups comprising from 12 to 21 carbon atoms, for example tallow fatty acid derivatives, R₁₄ denotes a methyl group, and R₁₅ denotes a hydrogen atom. Such a product is sold, for example, under the name Rewoquat^{®} W 75 by the company Rewo;
   ▪ - di- or triquaternary ammonium salts, in particular of formula (A5) below:
   ▪ 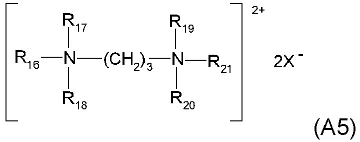
   ▪ in which formula (A5):
      ▪ R16 denotes an alkyl group comprising approximately from 16 to 30 carbon atoms, which is optionally hydroxylated and/or interrupted with one or more oxygen atoms;
      ▪ R17 is chosen from hydrogen, an alkyl group comprising from 1 to 4 carbon atoms or a group -(CH₂)₃-N⁺(R16a)(R17a)(R18a), X⁻;
      ▪ R16a, R17a, R18a, R18, R19, R20 and R21, which may be identical or different, are chosen from hydrogen and an alkyl group comprising from 1 to 4 carbon atoms; and
      ▪ X⁻, which may be identical or different, represent an organic or inorganic anionic counterion, such as that chosen from halides, acetates, phosphates, nitrates, (C₁-C₄)alkyl sulfates, (C₁-C₄)alkyl- or (C₁-C₄)alkylarylsulfonates, in particular methyl sulfate and ethyl sulfate.
      ▪ Such compounds are, for example, Finquat CT-P, provided by Finetex (Quaternium 89), or Finquat CT, provided by Finetex (Quaternium 75);
      ▪ - quaternary ammonium salts containing one or more ester functions, such as those of formula (A6) below:
   ▪ 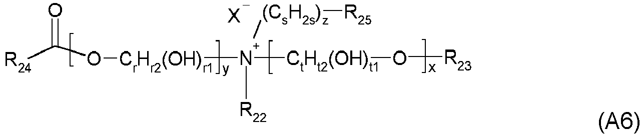
   ▪ in which formula (A6):
      ▪ R22 is chosen from C₁-C₆ alkyl and C₁-C₆ hydroxyalkyl or dihydroxyalkyl groups,
      ▪ R₂₃ is selected from:
   ▪ - the group 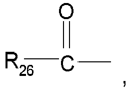
   ▪ - linear or branched, saturated or unsaturated C₁-C₂₂ hydrocarbon-based groups R27,
   ▪ - a hydrogen atom,
   ▪ R25 is selected from:
   ▪ - the group 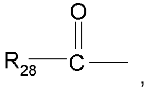
   ▪ - the groups R29, which are linear or branched, saturated or unsaturated C₁-C₆ hydrocarbon-based radicals;
   ▪ - a hydrogen atom,
   ▪ R24, R26 and R28, which are identical or different, are selected from linear or branched, saturated or unsaturated C₇-C₂₁ hydrocarbon radicals;
   ▪ r, s and t, which may be identical or different, are integers ranging from 2 to 6,
   ▪ r1 and t1, which may be identical or different, are equal to 0 or 1, with r2+r1=2r and t1+t2=2t,
   ▪ y is an integer ranging from 1 to 10,
   ▪ x and z, which may be identical or different, are integers ranging from 0 to 10,
   ▪ X⁻ represents an organic or inorganic anionic counterion,
   with the proviso that the sum x + y + z equals from 1 to 15, that, when x is 0, then R₂₃ denotes R₂₇ and that, when z is 0, then R₂₅ denotes R₂₉.

The alkyl groups R₂₂ may be linear or branched, and more particularly linear.

Preferably, R₂₂ denotes a methyl, ethyl, hydroxyethyl or dihydroxypropyl group, and more particularly a methyl or ethyl group.

Advantageously, the sum x + y + z is from 1 to 10.

When R₂₃ is an R₂₇ hydrocarbon group, it may be long and may contain from 12 to 22 carbon atoms, or may be short and may have from 1 to 3 carbon atoms.

When R₂₅ is an R₂₉ hydrocarbon-based group, it preferably contains 1 to 3 carbon atoms.

Advantageously, R₂₄, R₂₆ and R₂₈, which are identical or different, are selected from linear or branched, saturated or unsaturated C₁₁-C₂₁, hydrocarbon-based groups, and more particularly from linear or branched, saturated or unsaturated C₁₁-C₂₁, alkyl and alkenyl groups.

Preferably, x and z, which may be identical or different, are equal to 0 or 1.

Advantageously, y is equal to 1.

Preferably, r, s and t, which may be identical or different, equal 2 or 3, and even more particularly are equal to 2.

The anionic counterion X⁻ is preferably a halide, such as chloride, bromide or iodide; a (C₁-C₄)alkyl sulfate or a (C₁-C₄)alkyl- or (C₁-C₄)alkylarylsulfonate. However, it is possible to use methanesulfonate, phosphate, nitrate, tosylate, an anion derived from an organic acid, such as acetate or lactate, or any other anion that is compatible with the ammonium containing an ester function.

The anionic counterion X⁻ is even more particularly chloride, methyl sulfate or ethyl sulfate.

Use is made more particularly in the composition according to the invention of the ammonium salts of formula (A6) in which:
- R₂₂ denotes a methyl or ethyl group,
- x and y are equal to 1,
- z is equal to 0 or 1,
- r, s and t are equal to 2,
- R₂₃ is chosen from:
   - the group 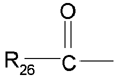
   - methyl, ethyl or C₁₄-C₂₂ hydrocarbon-based groups
   - a hydrogen atom,
- R₂₅ is chosen from:
   - the group 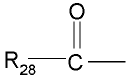
   - a hydrogen atom,
- R₂₄, R₂₆ and R₂₈, which may be identical or different, are chosen from linear or branched, saturated or unsaturated C₁₃-C₁₇ hydrocarbon-based groups, and preferably from linear or branched, saturated or unsaturated C₁₃-C₁₇ alkyl and alkenyl groups.

Advantageously, the hydrocarbon radicals are linear.

Among the compounds of formula (A6), examples that may be mentioned include salts, especially the chloride or methyl sulfate of diacyloxyethyldimethylammonium, diacyloxyethylhydroxyethylmethylammonium, monoacyloxyethyldihydroxyethylmethylammonium, triacyloxyethylmethylammonium or monoacyloxyethylhydroxyethyldimethylammonium, and mixtures thereof. The acyl groups preferably contain 14 to 18 carbon atoms and are obtained more particularly from a plant oil, such as palm oil or sunflower oil. When the compound contains several acyl groups, these groups may be identical or different.

These products are obtained, for example, by direct esterification of triethanolamine, triisopropanolamine, an alkyldiethanolamine or an alkyldiisopropanolamine, which are optionally oxyalkylenated, with fatty acids or with fatty acid mixtures of plant or animal origin, or by transesterification of the methyl esters thereof. This esterification is followed by a quaternization by means of an alkylating agent such as an alkyl halide, preferably methyl or ethyl halide, a dialkyl sulfate, preferably dimethyl or diethyl sulfate, methyl methanesulfonate, methyl *para-*toluenesulfonate, glycol chlorohydrin or glycerol chlorohydrin.

Such compounds are sold, for example, under the names Dehyquart^{®} by the company Henkel, Stepanquat^{®} by the company Stepan, Noxamium^{®} by the company Ceca or Rewoquat^{®} WE 18 by the company Rewo-Witco.

The composition according to the invention may contain, for example, a mixture of quaternary ammonium salts of monoesters, diesters and triesters with a weight majority of diester salts.

It is also possible to use the ammonium salts containing at least one ester function that are described in patents US-A-4 874 554 and US-A-4 137 180.

Use may be made of behenoylhydroxypropyltrimethylammonium chloride sold by KAO under the name Quatarmin BTC 131.

Preferably, the ammonium salts containing at least one ester function contain two ester functions.

Among the cationic surfactants that may be present in the composition it is more particularly preferred to choose cetyltrimethylammonium, behenyltrimethylammonium and dipalmitoylethyl-hydroxyethylmethylammonium salts, and mixtures thereof, and more particularly behenyltrimethylammonium chloride, cetyltrimethylammonium chloride, and dipalmitoylethylhydroxyethylammonium methosulfate, and mixtures thereof.

Examples of nonionic surfactants that may be used in the composition used are described, for example, in the Handbook of Surfactants by M.R. Porter, published by Blackie & Son (Glasgow and London), 1991, pp. 116-178. They are especially chosen from alcohols, α-diols and (C₁-C₂₀)alkylphenols, these compounds being polyethoxylated, polypropoxylated or polyglycerolated, and containing at least one fatty chain comprising, for example, from 8 to 40 carbon atoms, it being possible for the number of ethylene oxide and/or propylene oxide groups to especially range from 2 to 200, and for the number of glycerol groups to especially range from 2 to 30.

Mention may also be made of copolymers of ethylene oxide and propylene oxide, optionally oxyethylenated sorbitan fatty acid esters, sucrose fatty acid esters, polyoxyalkylenated fatty acid esters, optionally oxyalkylenated alkyl polyglycosides, alkyl glucoside esters, derivatives of N-alkyl glucamine and of N-acyl methylglucamine, aldobionamides, oxyethylenated and/or oxypropylenated silicones and amine oxides.

The nonionic surfactants are chosen from monooxyalkylenated or polyoxyalkylenated and monoglycerolated or polyglycerolated nonionic surfactants. The oxyalkylene units are more particularly oxyethylene or oxypropylene units, or their combination, preferably oxyethylene units.

Examples of oxyalkylenated nonionic surfactants that may be mentioned include:
- oxyalkylenated (C₈-C₂₄)alkylphenols;
- saturated or unsaturated, linear or branched, oxyalkylenated C₈-C₃₀ alcohols;
- saturated or unsaturated, linear or branched, oxyalkylenated C₈-C₃₀ amides;
- esters of saturated or unsaturated, linear or branched, C₈-C₃₀ acids and of polyethylene glycols;
- polyoxyethylenated esters of saturated or unsaturated, linear or branched, C₈-C₃₀ acids and of sorbitol;
- saturated or unsaturated oxyethylenated vegetable oils;
- condensates of ethylene oxide and/or of propylene oxide, *inter alia,* alone or as mixtures;
- oxyethylenated and/or oxypropylenated silicones.

The surfactants contain a number of moles of ethylene oxide and/or of propylene oxide of between 1 and 100, preferably between 2 and 50 and preferably between 2 and 30. Advantageously, the nonionic surfactants do not comprise any oxypropylene units.

In accordance with one preferred embodiment of the invention, the oxyalkylenated nonionic surfactants are chosen from oxyethylenated C₈-C₃₀ alcohols comprising from 1 to 100 mol of ethylene oxide; polyoxyethylenated esters of linear or branched, saturated or unsaturated C₈-C₃₀ acids and of sorbitol comprising from 1 to 100 mol of ethylene oxide.

As examples of monoglycerolated or polyglycerolated nonionic surfactants, monoglycerolated or polyglycerolated C₈-C₄₀ alcohols are preferably used.

In particular, the monoglycerolated or polyglycerolated C₈-C₄₀ alcohols correspond to formula (A7) below:

R₂₉O-[CH₂-CH(CH₂OH)-O]ₘ-H (A7)

in which formula (A7):
▪ R₂₉ represents a linear or branched C₈-C₄₀ and preferably C₈-C₃₀ alkyl or alkenyl radical; and
▪ m represents a number ranging from 1 to 30 and preferably from 1 to 10.

As examples of compounds of formula (A7) that are suitable within the context of the invention, mention may be made of lauryl alcohol containing 4 mol of glycerol (INCI name: Polyglyceryl-4 Lauryl Ether), lauryl alcohol comprising 1.5 mol of glycerol, oleyl alcohol comprising 4 mol of glycerol (INCI name: Polyglyceryl-4 Oleyl Ether), oleyl alcohol comprising 2 mol of glycerol (INCI name: Polyglyceryl-2 Oleyl Ether), cetearyl alcohol comprising 2 mol of glycerol, cetearyl alcohol comprising 6 mol of glycerol, oleocetyl alcohol comprising 6 mol of glycerol, and octadecanol comprising 6 mol of glycerol.

The alcohol of formula (A7) may represent a mixture of alcohols in the same way that the value of m represents a statistical value, which means that, in a commercial product, several species of polyglycerolated fatty alcohols may coexist in the form of a mixture.

Among the monoglycerolated or polyglycerolated alcohols, it is more particularly preferred to use the C₈/C₁₀ alcohol containing 1 mol of glycerol, the C₁₀/C₁₂ alcohol containing 1 mol of glycerol and the C₁₂ alcohol containing 1.5 mol of glycerol.

In accordance with another embodiment of the invention, the nonionic surfactants are chosen from polyoxyalkylenated silicones and more particularly polyoxyalkylenated polydimethylsiloxanes in which the polyoxyalkylene group represents an alternating sequence of oxygen atoms and of linear or branched C₂ to C₁₀, preferably C₂ to C₆ and more preferentially C₂ and/or C₃ alkylene groups (ethylene and propylene, respectively), the number of alkylene groups being between 1 and 100, preferably between 5 and 50 and more preferentially between 10 and 40. Examples that will be mentioned are the silicones of PEG/PPG 18/18 dimethicone type sold under the name 5225C by Dow Corning.

Mention may also be made of alkyl/polyoxyalkylene mixed polydimethylsiloxanes in which the alkyl group is a linear or branched C₈ to C₃₀ and preferably C₁₂ to C₂₂ alkyl chain, and the polyoxyalkylene group is an alternating sequence of oxygen atoms and of linear or branched C₂ to C₁₀, preferably C₂ to C₆ and more preferentially C₂ and/or C₃ linear alkylene groups (ethylene and propylene, respectively), the number of alkylene groups being between 1 and 100, preferably between 2 and 30 and more preferentially between 5 and 15, the distribution of the groups being such that the ethylene groups are the more numerous. Examples that will be mentioned include the silicones of Cetyl PEG/PPG-10/1 dimethicone type as sold under the name Abil EM 90 by Evonik.

Preferably, the surfactant used in the composition of the invention is a monooxyalkylenated or polyoxyalkylenated, particularly monooxyethylenated or polyoxyethylenated, or monooxypropylenated or polyoxypropylenated, nonionic surfactant, or a combination thereof, more particularly monooxyethylenated or polyoxyethylenated.

Preferably, the surfactant(s) are chosen from nonionic surfactants or from anionic surfactants. More particularly, the surfactant(s) present in the composition are chosen from nonionic surfactants. Better still, the surfactant is a polyoxyethylenated silicone.

In the inverse emulsion of the invention, the amount of surfactant(s) in the composition preferably ranges from 0.1% to 50% by weight and better still from 0.5% to 30% by weight relative to the total weight of the emulsion.

The emulsion may also contain various adjuvants conventionally used in hair treatment compositions, such as anionic, cationic, nonionic, amphoteric or zwitterionic polymers or mixtures thereof; antioxidants; penetrants; sequestrants; fragrances; dispersants; film-forming agents; preserving agents; opacifiers; dyes.

The above adjuvants are generally present in an amount for each of them of between 0.01% and 20% by weight relative to the weight of the emulsion. Advantageously, the inverse emulsion according to the invention may contain organic or mineral salts, preferably mineral salts of monovalent or divalent metal cations and in particular of sodium or magnesium, such as sodium chloride or magnesium sulfate. Preferably, the concentration of organic or mineral metal salts may range from 0.01% to 20%, better still from 0.1% to 10% and even better still from 0.5% to 5% by weight relative to the weight of the emulsion.

The emulsion may comprise one or more fumed silicas.

The fumed silicas may be obtained by high-temperature hydrolysis of a volatile silicon compound in an oxyhydrogen flame, producing a finely divided silica. This process makes it possible especially to obtain hydrophilic silicas having a large number of silanol groups at their surface. Such hydrophilic silicas are sold, for example, under the names Aerosil 130^{®}, Aerosil 200^{®}, Aerosil 255^{®}, Aerosil 300^{®} and Aerosil 380^{®} by the company Degussa, and Cab-O-Sil HS-5^{®}, Cab-O-Sil EH-5^{®}, Cab-O-Sil LM-130^{®}, Cab-O-Sil MS-55^{®} and Cab-O-Sil M-5^{®} by the company Cabot.

It is possible to chemically modify the surface of the silica via chemical reaction in order to reduce the number of silanol groups. It is possible in particular to replace silanol groups with hydrophobic groups: a hydrophobic silica is then obtained.

The hydrophobic groups can be:
- trimethylsiloxyl groups, which are obtained in particular by treating fumed silica in the presence of hexamethyldisilazane. Silicas thus treated are known as "Silica silylate" according to the CTFA (6th edition, 1995). They are sold, for example, under the references Aerosil R812^{®} by Degussa and Cab-O-Sil TS-530^{®} by Cabot.
- dimethylsilyloxyl or polydimethylsiloxane groups, which are obtained in particular by treating fumed silica in the presence of polydimethylsiloxane or dimethyldichlorosilane. Silicas thus treated are known as "Silica dimethyl silylate" according to the CTFA (6th edition, 1995). They are sold, for example, under the references Aerosil R972^{®} and Aerosil R974^{®} by Degussa and Cab-O-Sil TS-610^{®} and Cab-O-Sil TS-720^{®} by Cabot.

The fumed silica preferably has a particle size that may be nanometric to micrometric, for example ranging from about 5 to 200 nm.

When it is present, the fumed silica represents from 1% to 30% by weight relative to the weight of the emulsion.

The emulsion may also comprise one or more organic thickeners.

These thickeners may be chosen from fatty acid amides (coconut diethanolamide or monoethanolamide, oxyethylenated alkyl ether carboxylic acid monoethanolamide), polymeric thickeners such as cellulose-based thickeners (hydroxyethyl cellulose, hydroxypropyl cellulose, carboxymethyl cellulose), guar gum and derivatives thereof (hydroxypropyl guar).

The content of organic thickener(s), if they are present, usually ranges from 0.01% to 20% by weight and preferably from 0.1% to 5% by weight relative to the weight of the emulsion.

The emulsion of the invention comprises water and optionally one or more organic solvents.

Examples of organic solvents that may be mentioned include linear or branched and preferably saturated monoalcohols, comprising 2 to 6 carbon atoms, such as ethanol or isopropanol; aromatic alcohols such as benzyl alcohol or phenylethyl alcohol; polyols containing more than two hydroxyl functions, such as glycerol; polyol ethers, for instance ethylene glycol monomethyl, monoethyl or monobutyl ether, propylene glycol or ethers thereof, for instance propylene glycol monomethyl ether; and also diethylene glycol alkyl ethers, especially C₁-C₄ alkyl ethers, for instance diethylene glycol monoethyl ether or monobutyl ether, alone or as a mixture.

The organic solvents, when they are present, generally represent between 1% and 40% by weight relative to the total weight of the emulsion and preferably between 5% and 30% by weight relative to the total weight of the emulsion.

In one variant, the water concentration may range from 5% to 95%, better still from 10% to 90% and even better still from 15% to 50% of the total weight of the emulsion.

The inverse emulsion according to the invention may be in various forms, such as in the form of milks or creams, or in any other form that is suitable for treating keratin fibres, and especially human hair.

Preferably, the emulsion is in the form of a milk or a cream.

The pH of the emulsion according to the invention and/or of its aqueous phase is advantageously between 1 and 9 and preferably between 1.5 and 6, preferentially between 1.8 and 5, limits included.

It may be adjusted to the desired value by means of acidifying or basifying agents usually used in the dyeing of keratin fibres.

For a basifying agent, this agent may be chosen from mineral or organic or hybrid alkaline agents, or mixtures thereof.

The mineral alkaline agent(s) are preferably chosen from aqueous ammonia, alkali carbonates or bicarbonates such as sodium or potassium carbonates and sodium or potassium bicarbonates, sodium hydroxide or potassium hydroxide, or mixtures thereof.

The organic alkaline agent(s) are preferably chosen from organic amines with a pK_{b} at 25°C of less than 12, preferably less than 10 and even more advantageously less than 6. It should be noted that it is the pK_{b} corresponding to the function of highest basicity.

Mention may be made, as hybrid compounds, of the salts of the abovementioned amines with acids, such as carbonic acid or hydrochloric acid.

The organic alkaline agent(s) are chosen, for example, from amine derivatives such as alkanolamines, oxyethylenated and/or oxypropylenated ethylenediamines, amino acids of amines such as a 1,3-diaminopropane, 1,3-diamino-2-propanol, spermine or spermidine.

The term "alkanolamine" means an organic amine comprising a primary, secondary or tertiary amine function, and one or more linear or branched C₁-C₈ alkyl groups bearing one or more hydroxyl radicals.

Alkanolamines such as monoalkanolamines, dialkanolamines or trialkanolamines comprising from one to three identical or different C₁-C₄ hydroxyalkyl radicals are in particular suitable for performing the invention.

Examples that may be mentioned include monoethanolamine, diethanolamine, triethanolamine, monoisopropanolamine, diisopropanolamine, N-dimethyl-aminoethanolamine, 2-amino-2-methyl-1-propanol, triisopropanolamine, 2-amino-2-methyl-1,3-propanediol, 3-amino-1,2-propanediol, 3-dimethylamino-1,2-propanediol and tris(hydroxymethylamino)methane.

Examples of acidifying agents that may be mentioned include mineral or organic acids, for instance hydrochloric acid, orthophosphoric acid, carboxylic acids, for instance tartaric acid or lactic acid, or sulfonic acids.

The inverse emulsion of the invention is used by application to wet or dry keratin fibres, preferably after the application of a shampoo and/or a hair conditioner.

After a leave-in time ranging from one minute to one hour, preferably from 20 minutes to 45 minutes, optionally with the aid of a heating appliance, optionally under a protective film, the human keratin fibres are optionally rinsed with water, optionally washed with a shampoo and then rinsed with water, before being dried or left to dry. The drying may be performed naturally or, alternatively, using a hairdryer. It may be followed by blow-drying (brushing involving traction of the keratin fibres under a stream of hot air), and/or straightening using flat heating tongs.

Preferably, the hair is dried, blow-drying is performed and the hair is then straightened with flat heating tongs.

The temperature of the tongs is then between 100 and 250°C, preferably between 150 and 230°C and more particularly between 180 and 220°C.

The examples that follow serve to illustrate the invention without, however, being limiting in nature.

### EXAMPLES

### EXAMPLE 1 - INVERSE EMULSIONS BASED ON SILICONE OILS

The following emulsions according to the invention are prepared (the amounts are expressed in g% of starting material as supplied):

| | EMULSION 1A | EMULSION 1B |
|---|---|---|
| Cyclopentadimethylsiloxane (Mirasil CM5 - Bluestar) | 20 | - |
| Linear polydimethylsiloxane (viscosity: 10 cSt) Belsil DM 10 - Wacker | - | 20 |
| Oxyethylenated (10 OE) oleyl alcohol (Oleth-10) sold under the name Brij O10-SS-(MV) by the company Croda | 6 | 6 |
| Glyoxylic acid (50%) | 10 | 10 |
| Water | qs 100 | qs 100 |
| Sodium hydroxide | qs pH 3 | qs pH 3 |

### EXAMPLE 2 - INVERSE EMULSIONS BASED ON ESTERS

The following emulsions according to the invention are prepared (the amounts are expressed in g% of starting material as supplied):

| | EMULSION 2A | EMULSION 2B |
|---|---|---|
| Isopropyl myristate | 10 | 10 |
| Cetyl PEG/PPG-10/1 dimethicone sold under the reference Abil EM 90 by Evonik | 1 | 1 |
| Glyoxylic acid (50%) | 10 | 10 |
| Water | qs 100 | qs 100 |
| Sodium chloride | 1 | 1 |
| Sodium hydroxide | qs pH 2 | qs pH 3 |

### EXAMPLE 3 - INVERSE EMULSIONS BASED ON MINERAL OILS

The following emulsions according to the invention are prepared (the amounts are expressed in g% of starting material as supplied):

| | EMULSION 3A | EMULSION 3B |
|---|---|---|
| Isododecane | 10 | |
| Undecane | | 7 |
| Tridecane | | 3 |
| Cetyl PEG/PPG-10/1 dimethicone | 1 | 1 |
| Glyoxylic acid (50%) | 10 | 10 |
| Water | qs 100 | qs 100 |
| Sodium chloride | 1 | 1 |
| Sodium hydroxide | qs pH 2 | qs pH 3 |

### EXAMPLE 4 - INVERSE EMULSIONS WITH CATIONIC CO-SURFACTANT

The following emulsions according to the invention are prepared (the amounts are expressed in g% of starting material as supplied):

| | EMULSION 4A | EMULSION 4B |
|---|---|---|
| Isopropyl myristate | 10 | 10 |
| Cetyl PEG/PPG-10/1 dimethicone | 1 | 1 |
| Glyoxylic acid (50%) | 10 | 10 |
| Water | qs 100 | qs 100 |
| Sodium chloride | 1 | 1 |
| Behenyltrimethylammonium chloride | 2.4 | |
| Cetyltrimethylammonium chloride | | 1 |
| Isopropanol | 0.6 | |
| Sodium hydroxide | qs pH 2 | qs pH 3 |

### Mode of application

The emulsions are then applied to locks of natural Caucasian hair, at a rate of 2 g per 1 g of hair.

The mixtures are left to stand on the locks at room temperature for 20 minutes.

The hair is then dried, blow-dried, straightened 13 times using flat tongs brought to 230°C, washed with a standard shampoo and dried again. The shampooing and drying are repeated five times to demonstrate the remanence of the effect.

### Results

The volume of the locks after 24 hours at 25°C in an atmosphere of controlled humidity (76% humidity) is then observed. The untreated locks and the locks treated with the compositions described above are thus compared. Visually, the volume of the treated locks is less than that of the untreated locks.

Moreover, after the successive shampoo washes, the hair is shinier, feels softer and is easier to comb.

## Claims

1. Hair treatment process which comprises the application of an inverse emulsion (water-in-oil) comprising (a) at least 5% by weight of one or more liquid fatty substances relative to the total weight of the emulsion, (b) one or more non ionic surfactants and (c) one or more dicarbonyl compounds other than the compounds (a) which are an α-keto acid or a hydrate thereof, chosen from glyoxylic acid, pyruvic acid, oxaloacetic acid, ketoglutaric acid.

2. Process according to Claim 1, in which the liquid fatty substance(s) are chosen from liquid fatty alcohols, liquid fatty esters, silicone oils, C₆-C₁₆ hydrocarbons, hydrocarbons containing more than 16 carbon atoms, non-silicone oils of animal origin, plant oils of triglyceride type, synthetic triglycerides, fluoro oils and non-salified fatty acids.

3. Process according to either of the preceding claims, in which the fatty substance is chosen from silicone oils and liquid fatty alcohols.

4. Process according to Claim 1 or 2, in which the fatty alcohol(s) are chosen from alcohols of formula R-OH, in which R preferably denotes a C12-C24 branched alkyl or C12-C24 alkenyl group.

5. Process according to any one of the preceding claims, in which the liquid fatty substance is a silicone, preferably non-volatile polydialkylsiloxanes or polyorganosiloxanes.

6. Process according to any one of the preceding claims, in which the liquid fatty substance is included in an amount of between 5% and 90% and preferably between 7% and 20% by weight relative to the total weight of the emulsion.

7. Process according to any one of the preceding claims, in which the dicarbonyl compound is an α-keto acid or a hydrate thereof, chosen from glyoxylic acid and pyruvic acid.

8. Process according to any one of the preceding claims, in which the non ionic surfactant(s) are chosen from monooxyalkylenated or polyoxyalkylenated, particularly monooxyalkylenated or polyoxyalkylenated, and monoglycerolated or polyglycerolated non-ionic surfactants .

9. Process according to any one of the preceding claims, in which the nonionic surfactant is chosen from monooxyalkylenated or polyoxyalkylenated, particularly monooxyethylenated or polyoxyethylenated, and monooxypropylenated or polyoxypropylenated surfactants, or a combination thereof, more particularly monooxyethylenated or polyoxyethylenated surfactants, preferably a polyoxyethylenated silicone or a polyoxyalkylenated fatty alcohol.

10. Process according to any one of the preceding claims, moreover comprising one or more cationic surfactants, preferably bearing an alkylammonium group.

11. Process according to any one of the preceding claims, moreover comprising a step of straightening the hair, preferably at a temperature above 100°C.

12. Inverse emulsion (water-in-oil) comprising (a) at least 5% by weight of one or more liquid fatty substances relative to the total weight of the emulsion, (b) one or more non-ionic surfactants chosen from monooxyalkylenated or polyoxyalkylenated, particularly monooxyalkylenated or polyoxyalkylenated, and monoglycerolated or polyglycerolated non-ionic surfactants and (c) one or more dicarbonyl compounds other than the compounds (a) which is an α-keto acid or a hydrate thereof, chosen from glyoxylic acid, pyruvic acid, oxaloacetic acid, ketoglutaric acid.

13. Emulsion according to claim 12 further defined according to any one of claims 2 to 10.

## Patentansprüche

1. Haarbehandlungsverfahren, das das Aufbringen einer inversen Emulsion (Wasser-in-Öl), umfassend (a) mindestens 5 Gew.-% einer oder mehrerer flüssiger Fettsubstanzen, bezogen auf das Gesamtgewicht der Emulsion, (b) ein oder mehrere nichtionische Tenside und (c) eine oder mehrere Dicarbonylverbindungen, die von den Verbindungen (a) verschieden sind, bei denen es sich um eine α-Ketosäure oder ein Hydrat davon, ausgewählt aus Glyoxylsäure, Brenztraubensäure, Oxalessigsäure und Ketoglutarsäure, handelt, umfasst.

2. Verfahren nach Anspruch 1, wobei die flüssige Fettsubstanz bzw. flüssigen Fettsubstanzen aus flüssigen Fettalkoholen, flüssigen Fettsäureestern, Silikonölen, C₆-C₁₆-Kohlenwasserstoffen, Kohlenwasserstoffen mit mehr als 16 Kohlenstoffatomen, Nichtsilikonölen tierischer Herkunft, Pflanzenölen vom Triglycerid-Typ, synthetischen Triglyceriden, Fluorölen und nicht versalzten Fettsäuren ausgewählt ist bzw. sind.

3. Verfahren nach einem der vorhergehenden Ansprüche, wobei die Fettsubstanz aus Silikonölen und flüssigen Fettalkoholen ausgewählt ist.

4. Verfahren nach Anspruch 1 oder 2, wobei der Fettalkohol bzw. die Fettalkohole aus Alkoholen der Formel R-OH, in der R vorzugsweise für eine verzweigte C₁₂-C₂₄-Alkyl- oder C₁₂-C₂₄-Alkenylgruppe steht, ausgewählt ist bzw. sind.

5. Verfahren nach einem der vorhergehenden Ansprüche, wobei es sich bei der flüssigen Fettsubstanz um ein Silikon, vorzugsweise nichtflüchtige Polydialkylsiloxane oder Polyorganosiloxane, handelt.

6. Verfahren nach einem der vorhergehenden Ansprüche, wobei die flüssige Fettsubstanz in einer Menge zwischen 5 und 90 Gew.-% und vorzugsweise zwischen 7 und 20 Gew.-%, bezogen auf das Gesamtgewicht der Emulsion, enthalten sind.

7. Verfahren nach einem der vorhergehenden Ansprüche, wobei es sich bei der Dicarbonylverbindung um eine α-Ketosäure oder ein Hydrat davon, ausgewählt aus Glyoxylsäure und Brenztraubensäure, handelt.

8. Verfahren nach einem der vorhergehenden Ansprüche, wobei das nichtionische Tensid bzw. die nichtionischen Tenside aus monooxyalkylenierten oder polyoxyalkylenierten, insbesondere monooxyalkylenierten oder polyoxyalkylenierten, und monoglycerinierten oder polyglycerinierten nichtionischen Tensiden ausgewählt ist bzw. sind.

9. Verfahren nach einem der vorhergehenden Ansprüche, wobei das nichtionische Tensid aus monooxyalkylenierten oder polyoxyalkylenierten, insbesondere monooxyethylenierten oder polyoxyethylenierten und monooxypropylenierten oder polyoxypropylenierten, Tensiden oder einer Kombination davon, spezieller monooxyethylenierten oder polyoxyethylenierten Tensiden, vorzugsweise einem polyoxyethylenierten Silikon oder einem polyoxyalkylenierten Fettalkohol, ausgewählt ist.

10. Verfahren nach einem der vorhergehenden Ansprüche, außerdem umfassend ein oder mehrere kationische Tenside, vorzugsweise mit einer Alkylammoniumgruppe.

11. Verfahren nach einem der vorhergehenden Ansprüche, außerdem umfassend einen Schritt des Glättens des Haars, vorzugsweise bei einer Temperatur über 100 °C.

12. Inverse Emulsion (Wasser-in-Öl), umfassend (a) mindestens 5 Gew.-% einer oder mehrerer flüssiger Fettsubstanzen, bezogen auf das Gesamtgewicht der Emulsion, (b) ein oder mehrere nichtionische Tenside, ausgewählt aus monooxyalkylenierten oder polyoxyalkylenierten, insbesondere monooxyalkylenierten oder polyoxyalkylenierten, und monoglycerinierten oder polyglycerinierten nichtionischen Tensiden, und (c) eine oder mehrere Dicarbonylverbindungen, die von den Verbindungen (a) verschieden sind, bei denen es sich um eine α-Ketosäure oder ein Hydrat davon, ausgewählt aus Glyoxylsäure, Brenztraubensäure, Oxalessigsäure und Ketoglutarsäure, handelt.

13. Emulsion nach Anspruch 12, ferner definiert gemäß einem der Ansprüche 2 bis 10.

## Revendications

1. Procédé de traitement capillaire qui comprend l'application d'une émulsion inverse (eau-dans-huile) comprenant (a) au moins 5 % en poids d'une ou plusieurs substances grasses liquides par rapport au poids total de l'émulsion, (b) un ou plusieurs tensioactifs non ioniques et (c) un ou plusieurs composés de type dicarbonyle différents des composés (a) qui sont un α-cétoacide ou un hydrate correspondant, choisi parmi l'acide glyoxylique, l'acide pyruvique, l'acide oxaloacétique, l'acide cétoglutarique.

2. Procédé selon la revendication 1, dans lequel la ou les substances grasses liquides sont choisies parmi des alcools gras liquides, des esters gras liquides, des huiles de silicone, des hydrocarbures en C₆₋₁₆, des hydrocarbures contenant plus de 16 atomes de carbone, des huiles non siliconées d'origine animale, des huiles végétales du type triglycéride, des triglycérides synthétiques, des huiles fluorées et des acides gras non salifiés.

3. Procédé selon l'une ou l'autre des revendications précédentes, dans lequel la substance grasse est choisie parmi des huiles de silicone et des alcools gras liquides.

4. Procédé selon la revendication 1 ou 2, dans lequel l'alcool gras ou les alcools gras sont choisis parmi des alcools de formule R-OH, dans laquelle R désigne préférablement un groupe alkyle ramifié en C12 à C24 ou un groupe alcényle en C12 à C24.

5. Procédé selon l'une quelconque des revendications précédentes, dans lequel la substance grasse liquide est une silicone, préférablement des polydialkylsiloxanes ou polyorganosiloxanes non volatils.

6. Procédé selon l'une quelconque des revendications précédentes, dans lequel la substance grasse liquide est comprise en une quantité comprise entre 5 % et 90 % et préférablement entre 7 % et 20 % en poids par rapport au poids total de l'émulsion.

7. Procédé selon l'une quelconque des revendications précédentes, dans lequel le composé de type dicarbonyle est un α-cétoacide ou un hydrate correspondant, choisi parmi l'acide glyoxylique et l'acide pyruvique.

8. Procédé selon l'une quelconque des revendications précédentes, dans lequel le ou les tensioactifs non ioniques sont choisis parmi des tensioactifs non ioniques monooxyalkylénisés ou polyoxyalkylénisés, particulièrement parmi des tensioactifs non ioniques monooxyalkylénisés ou polyoxyalkylénisés, et monoglycérolés ou polyglycérolés.

9. Procédé selon l'une quelconque des revendications précédentes, dans lequel le tensioactif non ionique est choisi parmi des tensioactifs monooxyalkylénisés ou polyoxyalkylénisés, particulièrement monooxyéthylénisés ou polyoxyéthylénisés, et monooxypropylénisés ou polyoxypropylénisés, ou une combinaison correspondante, plus particulièrement des tensioactifs monooxyéthylénisés ou polyoxyéthylénisés, préférablement une silicone polyoxyéthylénisée ou un alcool gras polyoxyalkylénisé.

10. Procédé selon l'une quelconque des revendications précédentes, comprenant en outre un ou plusieurs tensioactifs cationiques, portant préférablement un groupe alkylammonium.

11. Procédé selon l'une quelconque des revendications précédentes, comprenant en outre une étape de lissage des cheveux, préférablement à une température supérieure à 100 °C.

12. Émulsion inverse (eau-dans-huile) comprenant (a) au moins 5 % en poids d'une ou plusieurs substances grasses liquides par rapport au poids total de l'émulsion, (b) un ou plusieurs tensioactifs non ioniques choisis des tensioactifs non ioniques monooxyalkylénisés ou polyoxyalkylénisés, particulièrement parmi des tensioactifs non ioniques monooxyalkylénisés ou polyoxyalkylénisés, et monoglycérolés ou polyglycérolés et (c) un ou plusieurs composés de type dicarbonyle différents des composés (a) qui est un α-cétoacide ou un hydrate correspondant, choisi parmi l'acide glyoxylique, l'acide pyruvique, l'acide oxaloacétique, l'acide cétoglutarique.

13. Émulsion selon la revendication 12 en outre définie selon l'une quelconque des revendications 2 à 10.
